# EUROPEAN PATENT SPECIFICATION

(11) **EP 4 185 135 B1**
(45) Date of publication and mention of the grant of the patent: **04.09.2024**
(21) Application number: 21739376.8
(22) Date of filing: 05.07.2021
(51) Int. Cl.: A24F 40/20, A24F 40/40

(54) **AEROSOL GENERATING DEVICE WITH ODOUR INHIBITION ASSEMBLY**
AEROSOLERZEUGUNGSVORRICHTUNG MIT GERUCHSHEMMUNGSANORDNUNG
DISPOSITIF DE GÉNÉRATION D'AÉROSOL DOTÉ D'UN ENSEMBLE D'INHIBITION D'ODEUR

(30) Priority: 22.07.2020 EP 20187216
(43) Date of publication of application: 31.05.2023
(73) Proprietor: Philip Morris Products S.A., 2000 Neuchâtel (CH)
(72) Inventor: ARAMENDY, Flavie, 2000 Neuchâtel (CH); CALI, Ricardo, 68163 Mannheim (DE); SALVADOR, Thomas Philipp, 68163 Mannheim (DE)
(74) Representative: Reddie & Grose LLP
(86) International application number: PCT/EP2021/068534
(87) International publication number: WO 2022/017764

(56) References cited:
- EP-A1- 3 491 945
- WO-A2-2018/206615

## Description

The present invention relates to an aerosol generating device for use with an aerosol generating article. In particular, the present invention relates to an aerosol generating device for use with an aerosol generating article, the aerosol generating device comprises an odour inhibition assembly.

Aerosol generating articles in which an aerosol-forming substrate, such as a tobacco containing substrate, is heated rather than combusted are known in the art. Typically, in such aerosol generating articles an aerosol is generated by the transfer of heat from a heat source to a physically separate aerosol-forming substrate or material, which may be located in contact with, within, around, or downstream of the heat source. During use of the aerosol generating article, volatile compounds are released from the aerosol-forming substrate by heat transfer from the heat source and are entrained in air drawn through the aerosol generating article. As the released compounds cool, they condense or nucleate to form an aerosol.

A number of prior art documents disclose aerosol-generating devices for consuming aerosol generating articles. Such devices include, for example, electrically heated aerosol generating devices in which an aerosol is generated by the transfer of heat from one or more electrical heater elements of the aerosol-generating device to the aerosol-forming substrate of a heated aerosol generating article.

However, it has been found that the process of heating the aerosol-forming substrate can also lead to the generation of unpleasant odours which is undesirable. This problem may be particularly pronounced when the aerosol-forming substrate comprises tobacco. In some instances, it has been observed that the aerosol generated by the aerosol generating device remains in the air and the surrounding environment after the user has finished using the aerosol generating device. This is known as *"off-odour".* In other instances, it has been observed that improper use or maintenance of the aerosol generating device can lead to a distinct unpleasant odour. This may occur when, for example, an electric heater element of the aerosol generating device is not properly cleaned. This is known as *"malodour".* Both off-odour and malodour may be experienced by both the user of the aerosol generating device and by others in close proximity to the aerosol generating device.

EP3491945A1 describes a flavour inhaler for improving the degree of freedom in controlling the amounts of an inhaled aerosol and a flavour. The flavour inhaler includes an aerosol source, an atomizing unit that atomizes the aerosol source and generates an aerosol, a flavour source provided downstream from the atomizing unit; a mouthpiece provided downstream from the flavour source; an aerosol flow path that guides the aerosol generated in the atomizing unit to the mouthpiece and that comprises a first flow path leading to the mouthpiece via the flavour source and a second flow path differing from the first flow path, the starting point of said second flow path being either directly or indirectly connected to the first flow path; and a flow rate adjustment mechanism that makes it possible to adjust the air flow rate ratio of the first flow path and the second flow path. The mouthpiece positioned downstream the flavour source is constructed to make the aerosol flow path to be opened toward the outside of the flavour inhaler. A user takes air including the aerosol into the mouth by holding the mouthpiece in the user's mouth and inhaling it.

Accordingly, it would be desirable to provide an aerosol generating device in which the unpleasant odours, such as off-odour and malodour, caused by the heating of the aerosol-forming substrate are reduced or prevented.

It may be desirable to provide an aerosol generating system where a user is able to control or customise the odour experienced by the user and by those around the aerosol generating device.

It may be desirable to provide an aerosol generating system which reduces or prevents unpleasant odours without the use of overpowering fragrances or pleasant odours.

The present invention relates to an aerosol generating device for use with an aerosol generating article. The aerosol generating article comprises an aerosol-forming substrate. The device comprises a heater for heating an aerosol-forming substrate. The device comprises an odour inhibition assembly.

The provision of an odour inhibition assembly may advantageously reduce or prevent unpleasant odours, such as off-odour and malodour, caused by the heating of the aerosol-forming substrate.

The odour inhibition assembly comprises at least one chamber for receiving at least one odour inhibition substrate.

The provision of an odour inhibition assembly comprising a chamber for receiving an odour inhibition substrate may allow a user to replace the odour inhibition substrate once it has been consumed. This may advantageously allow the odour inhibition assembly to be reusable. This may also allow a user to easily remove the odour inhibition substrate such that the user may be able to effectively temporarily deactivate the odour inhibition assembly if the user wished to do so.

According to the invention there is provided an aerosol generating device for use with an aerosol generating article comprising an aerosol-forming substrate. The device comprises a heater for heating an aerosol-forming substrate and an odour inhibition assembly. The odour inhibition assembly comprises at least one chamber for receiving at least one odour inhibition substrate. The device further comprises a recess for receiving an aerosol generating article, the at least one chamber being isolated from the recess such that air is unable to pass from the chamber to the recess through the aerosol generating device.

A user may use the aerosol generating device in cooperation with an aerosol generating article comprising an aerosol-forming substrate. The heater of the aerosol generating device heats the aerosol-forming substrate of the aerosol generating article to generate an aerosol which may be inhaled by a user. This may also release certain compounds which cause an unpleasant odour such as off-odour or malodour. The odour inhibition assembly may release at least one odour inhibition molecule to reduce or prevent these unpleasant odours. In particular, the at least one odour inhibition molecule may be released from at least odour inhibition substrate received in the at least one chamber of the odour inhibition assembly. As set out in more detail below, the odour inhibition substrate may release an odour inhibition molecule which comprises an odour masking molecule such as a fragrance. The odour inhibition substrate may release an odour inhibition molecule, or specific combination of odour inhibition molecules, which is configured to produce an olfactory white based on the odours generated by the heating of an aerosol generating article. The odour inhibition substrate may release a combination of an odour masking molecule and a molecule configured to produce an olfactory white. An olfactory white is neither pleasant nor malodourous.

The odour inhibition assembly releases the odour inhibition molecule into the air surrounding the aerosol generating device. In this way, any unpleasant odour in the air surrounding the aerosol generating device may be advantageously reduced or prevented. This may be beneficial both for the user of the aerosol generating device and for others in close proximity to the aerosol generating device.

As used herein with reference to the present invention, the term "aerosol generating article" refers to an article in which an aerosol-forming substrate is heated to produce and deliver inhalable aerosol to a consumer. As used herein, the term "aerosol-forming substrate" refers to a substrate capable of releasing volatile compounds upon heating to generate an aerosol.

A conventional cigarette is lit when a user applies a flame to one end of the cigarette and draws air through the other end. The localised heat provided by the flame and the oxygen in the air drawn through the cigarette causes the end of the cigarette to ignite, and the resulting combustion generates an inhalable smoke. By contrast, in heated aerosol generating articles, an aerosol is generated by heating an aerosol-forming substrate, such as tobacco. Known heated aerosol generating articles include, for example, electrically heated aerosol generating articles and aerosol generating articles in which an aerosol is generated by the transfer of heat from a combustible fuel element or heat source to a physically separate aerosol forming material. Heated aerosol generating articles also include vaping devices configured to generate an aerosol from a liquid aerosol-forming substrate.

The provision of the at least one chamber being isolated from the recess may prevent odour inhibition molecules from the odour inhibition assembly passing into the recess and being directly inhaled into the oral cavity of a user. As set out above, it is intended that that odour inhibition molecules are released into the surrounding air to reduce or prevent unpleasant odours originating from use of the aerosol generating device. It may not be desirable that the odour inhibition molecules interact directly with the mainstream aerosol generated within the aerosol generating device before both are inhaled into the oral cavity of a user. Isolating the at least one chamber from the recess may advantageously prevent this from happening.

The aerosol generating device may include air management which fully separates the airflow through the odour inhibition assembly from the airflow through the recess for receiving an aerosol generating article.

The aerosol generating device may be generally elongate in shape and extend between a proximal end and a distal end.

As used herein with reference to the present invention, the term "proximal end" refers to the end of the aerosol generating device closest to the recess for receiving an aerosol generating article. The "distal end" refers to the end of the aerosol generating device opposed to the "proximal end".

Where the aerosol generating device is generally elongate in shape and extends between a proximal end and a distal end, the direction between the proximal end and a distal end may be referred to as the "longitudinal axis" or the "longitudinal direction" of the aerosol generating device.

The odour inhibition assembly may be disposed anywhere on the device. For example, the odour inhibition assembly may be disposed between the proximal end and the distal end of the device. The odour inhibition assembly may be disposed closer to the proximal end of the device than to the distal end of the device; in other words the odour inhibition assembly may be located in the proximal half of the aerosol generating device. The odour inhibition assembly may be disposed at the proximal end of the device.

The odour inhibition assembly may be disposed closer to the distal end of the device than to the proximal end of the device; in other words the odour inhibition assembly may be located in the distal half of the aerosol generating device. The odour inhibition assembly may be disposed at the distal end of the device.

Locating the odour inhibition assembly at the distal end of the device may ensure the maximum distance between the odour inhibition assembly and the recess for receiving an aerosol generating article. This may advantageously further prevent the odour inhibition molecules of the odour inhibition assembly from passing into the recess for receiving an aerosol generating article and being directly inhaled into the oral cavity of a user.

Locating the odour inhibition assembly at the distal end of the device may advantageously help ensure the odour inhibition molecules from the odour inhibition assembly are distributed widely into the air surrounding the aerosol generating device to most effectively reduce or prevent unpleasant odours in the surrounding air. This may be particularly relevant for improving the experience for those around the aerosol generating device who are not users of the aerosol generating device.

The odour inhibition assembly may be located on the longitudinal surface of the aerosol generating device. The odour inhibition assembly may be located on a distal end face of the aerosol generating device. This may advantageously further ensure maximum separation of the odour inhibition assembly from the recess at the proximal end of the device.

The recess for receiving an aerosol generating article may be disposed on the longitudinal surface of the aerosol generating device. The recess for receiving an aerosol generating article may be located on a proximal end face of the aerosol generating device. This may advantageously further ensure maximum separation between the odour inhibition assembly and the recess.

The at least one chamber may be an open chamber with no closure.

The odour inhibition assembly may further comprise a cover movable between a closed position, in which the at least one chamber is closed by the cover, and an open position, in which the at least one chamber is not closed by the cover.

The provision of a cover may advantageously allow the odour inhibition substrate to be retained in the chamber during use, regardless of the orientation of the aerosol generating device. The provision of a cover may also prevent a user inadvertently touching the odour inhibition substrate when using the aerosol generating device. This may be advantageous for hygiene reasons and may also ensure the odour inhibition substrate is able to function effectively.

The provision of a cover movable between an open and closed position may allow a user to replace the odour inhibition substrate once it becomes consumed. This may advantageously allow the odour inhibition assembly to be reused.

The cover may be attached to the remainder of the aerosol generating device by any means. The cover may be attached to the remainder of the aerosol generating device by a sliding mechanism, or a pivot mechanism. The cover is advantageously attached to the remainder of the aerosol generating device by a hinge mechanism. The cover may be integrally formed with at least a portion of the remainder of the aerosol generating device; where this is the case, the hinge mechanism may be a living hinge.

The cover may be fully removable from the remainder of the aerosol generating device.

The odour inhibition assembly may comprise a retaining means for retaining the cover in the closed position. The retaining means may comprise a magnetic closure, a bolt, a clasp, a lock, a latch, or an interference fit.

The cover may comprise a window through which the at least one cavity may be visible. The provision of a window may advantageously allow a user to readily determine whether the at least one cavity contains an odour inhibition substrate. The window may further allow a user to check the state of an odour inhibition substrate within the at least one cavity.

The window may comprise a portion of transparent material. The cover may be entirely formed from transparent material.

The window may comprise a mesh portion through which the at least one cavity may be seen while still retaining an odour inhibition substrate where present.

Where the odour inhibition assembly comprises a cover, the odour inhibition assembly may comprise a means by which the odour inhibition molecules may pass out of the odour inhibition assembly when the cover is in the closed positon.

The odour inhibition assembly may comprise an airflow pathway configured such that air may pass into the assembly though an air inlet, pass through the at least one chamber, and pass out of an air outlet. In other words, the odour inhibition assembly provides a one-way airflow pathway.

The provision of a one-way airflow pathway may allow air to enter the odour inhibition assembly through an air inlet and pass into the at least one chamber where the air may become entrained with odour inhibition molecules from an odour inhibition substrate disposed in the at least one chamber. The air entrained with odour inhibition molecules may then pass out of the odour inhibition assembly through the air outlet such that the odour inhibition molecules may be dispersed into the air surrounding aerosol generating device.

The air inlet and air outlet are in fluid communication with the at least one chamber.

The provision of a one-way airflow pathway may advantageously maximise the concentration of odour inhibition molecules released into the ambient air.

The air inlet may comprise any number of openings. For example, the air inlet may comprise between 1 and 10 openings.

The air outlet may comprise any number of openings. For example, the air outlet may comprise between 1 and 10 openings.

The aerosol generating device may comprise a housing. At least one of the air inlet and the air outlet may comprise at least one opening provided through the housing of the aerosol generating device.

The odour inhibition assembly may further comprise a filter to reduce or prevent foreign objects entering the odour inhibition assembly. The filter may be disposed between the air inlet and the at least one chamber such that any air entering the odour inhibition assembly through the air inlet must pass through the filter. The filter may be a paper filter configured to remove fine foreign objects such as dust. The filter may comprise a mesh or gauze configured to remove only larger foreign objects.

The air outlet may be provided through the cover. The air outlet may comprise at least one opening provided through the cover.

The provision of an air outlet through the cover may advantageously provide a convenient way to get the air entrained with the odour inhibition molecules out of the chamber. As set out above, it may be advantageous to allow a user to see through the cover into the chamber. The provision of an air outlet in the cover may advantageously allow the user to see into the cavity through the air outlet.

The air outlet may comprise a plurality of elongate openings or slits.

The aerosol generating device may further comprise a charging port for connecting the aerosol generating device to an external power supply, wherein the charging port is in fluid communication with the at least one chamber, the charging port acting as the air inlet.

Where the aerosol generating device comprises an electric heater, the device may include charging port for connecting the aerosol generating device to an external power supply. The external power supply may be used to charge a rechargeable battery within the aerosol generating device. Alternatively, or in addition, the external power supply may supply power directly to the electric heater, and other electronic components of the aerosol generating device.

The charging port may be any charging port. For example, the charging port may be a micro-USB port or a mini-USB port.

The charging port may comprise a recess with electrical connections located in the recess. To allow the charging port to also act as an air inlet, the recess may comprise at least one opening within the recess, the at least one opening being in fluid communication with the chamber.

In use, air enters the recess of the charging port, passes through the at least one opening and into the chamber.

The provision of a charging port acting as an air inlet advantageously reduces the number of openings needed in the housing of the aerosol generating device. This may reduce manufacturing complexity and improve the appearance of the device. The provision of a charging port acting as an air inlet may be particularly suitable for devices which include an internal power supply which is charged by an external power supply. This would allow the charging port to be empty when the aerosol generating device is in use and the air inlet will advantageously not be blocked by an external power supply.

The charging port may be located anywhere on the aerosol generating device. Preferably, the charging port is located close to the odour inhibition assembly. This may advantageously limit the distance the air needs to travel from the air inlet to the at least one chamber.

The charging port may be located on the distal end face of the aerosol generating device. The charging port may be located adjacent the at least one chamber of the odour inhibition assembly. Where the at least one chamber comprises two chambers, the charging port may be disposed between the two chambers.

The cover may further comprise an opening configured to be aligned with the charging port when the cover is in the closed positon. This may be particularly advantageous where the charging port is located adjacent the at least one chamber, or where the charging port is disposed between two separate chambers since it allows the single cover to extend over, and close all chambers.

When the aerosol generating device is being connected to an external power supply, the external power supply passes through the opening in the cover and into the charging port of the aerosol generating device.

The opening also allows air to enter the charging port when the cover is in the closed position. The opening may also allow the aerosol generating device to be connected to an external power supply while the cover is in a closed positon. This advantageously means the device may be connected to an external power supply while still retaining any odour inhibition substrates in the at least one chamber.

The odour inhibition assembly may further comprise a means to ensure the air passes from the air inlet to the air outlet along the airflow pathway. For example, the odour inhibition assembly may comprise at least one valve along the airflow pathway to prevent air passing from the air outlet to the air inlet.

The odour inhibition assembly may further comprise a fan configured to move the air along the airflow pathway. The fan may move air from the air inlet to the air outlet. The provision of a fan may also advantageously force a greater concentration of odour inhibition molecules into the air surrounding the aerosol generating article. The fan may comprise a plurality of fan blades arranged to rotate about a central axis. The central axis of the fan may be aligned with the longitudinal axis of the aerosol generating device.

The fan may be located at any point along the airflow pathway. The fan may be arranged such that the air inlet is in communication with the centre of the fan and the air outlet is in communication with the outer region of the fan. Since the outer region of the fan blades move faster than the central region of the fan, this arrangement may advantageously allow air to be drawn in through the air inlet and forced out into the at least one chamber.

The odour inhibition assembly may comprise more than one fan.

The fan may be activated by a user input device, such as a button or a switch incorporated into the aerosol generating device. This may advantageously allow a user to increase the delivery of odour inhibition molecule if an unpleasant odour is detected.

The fan may be configured to be activated whenever the heater of the aerosol generating device is activated. This may advantageously ensure that the odour inhibition arrangement releases odour inhibition molecules when the heater heats an aerosol-forming substrate.

The fan may be configured to be activated by a puff detection means. In this case, the aerosol generating device comprises a puff detection sensor to detect where air is drawn through the aerosol generating device by a user. This may advantageously ensure that the odour inhibition arrangement releases odour inhibition molecules when aerosol from the heated aerosol-forming substrate is released.

The fan may be any size. For example, the fan may have a diameter of between about 5 millimetres and about 15 millimetres. The fan may have a diameter of about 10 millimetres.

The total depth of the fan, in a direction perpendicular to the fan's diameter, may be between about 1 millimetre and about 5 millimetres. The fan may have a total depth of about 2 millimetres.

The fan may be configured to be operated at any voltage. For example, the fan may be configured to be operated at between about 1 volt and about 5 volts. The fan may be configured to be operated at about 3.3 volts.

The fan may have a power consumption of between about 100 milliwatts and about 200 milliwatts. The fan may have a power consumption of about 165 milliwatts.

Alternately, or in addition, the odour inhibition assembly may comprise a pump to ensure the air passes from the air inlet to the air outlet along the airflow pathway. The pump may function in substantially the same way as the fan discussed above.

The fan may have any airflow. The fan may have an airflow of at least about 0.5 litres per minute. The airflow of at least about 0.5 litres per minute may advantageously ensure that sufficient airflow passes through the odour inhibition assembly to release the desired concentration of odour inhibition molecule.

The fan may have an airflow of at least about 1 litre per minute.

The fan may have an airflow of no more than about 3 litres per minute. The provision of a fan having an airflow of no more than about 3 litres per minute may advantageously prevent the odour inhibition molecules being dispersed too widely in the air surrounding the aerosol generating device.

The fan may have an airflow of between about 0.5 litres per minute and about 3 litres per minute. The fan may have an airflow of about 1.1 litres per minute.

The aerosol generating device may further comprise an internal power source configured to supply power to both the heater and the fan. The internal power source may comprise a battery. The internal power source may comprise a capacitor. The internal power source may be chargeable from an external power supply via the charging port.

The at least one chamber may comprise a porous support structure to allow air to enter the at least one chamber.

The provision of a chamber at least partially defined by a porous support structure may allow air to access all sides of an odour inhibition substrate disposed in the at least one chamber. This may advantageously maximise the concentration of odour inhibition molecules entrained in the air flowing through the odour inhibition assembly.

The porous support structure may accommodate at least one odour inhibition substrate. The porous support structure may be open on one side to allow access to the interior of the at least one chamber. The open side may be preferably aligned with the opening of the at least one chamber.

The porous support structure may comprise a wire mesh.

The porous support structure may be removable from the chamber. This may advantageously allow a user to clean the porous support structure which may be necessary after repeated contact with an odour inhibition substrate.

The at least one chamber may comprise a chamber having a thickness of at least about 2 millimetres.

As used herein, the "thickness" of the at least one chamber refers to the smallest dimension of the at least one chamber. Where the smallest dimension of the at least one chamber is aligned with the opening of the at least one chamber, the "thickness" may be referred to as the "depth" of the chamber. Where the at least one chamber is located on the distal end face of the aerosol generating device, and the smallest dimension of the at least one chamber is aligned with the opening of the at least one chamber, the "thickness" is will be the dimension of the at least one chamber aligned with the longitudinal axis of the aerosol generating device.

The size and shape of the at least one chamber may be selected to accommodate one or more odour inhibition substrates.

The at least one chamber may have a thickness of no more than about 10 millimetres. For example, the at least one chamber may have a thickness of no more than about 8 millimetres or no more than about 6 millimetres.

The at least one chamber may have a thickness of between about 2 millimetres and about 8 millimetres, or between about 2 millimetres and about 6 millimetres.

The size and shape of the chamber may be selected to accommodate at least 2, at least 3, at least 4, or at least 5 odour inhibition substrates. The at least one chamber may comprise a plurality of individual chambers, each configured to accommodate a single odour inhibition substrate.

The at least one chamber may comprise a first chamber for receiving a first odour inhibition substrate, and a second chamber for receiving a second odour inhibition substrate.

The provision of a first and a second chamber may provide an increased capacity in the at least one chamber. This may advantageously allow a greater number of odour inhibition substrates to be retained by the odour inhibition assembly. This may allow the odour inhibition assembly to be used more times before needing to replace the odour inhibition substrates. Furthermore, the provision of two chambers to receive two separate odour inhibition substrates may be preferable to providing one large chamber, even if that one large chamber has the same volume as the two smaller chambers combined. This is because two separate odour inhibition substrates may have a greater surface area than one larger odour inhibition substrate. A larger surface area of the odour inhibition substrate may advantageously increase the concentration of odour inhibition molecule entrained in the air passing through the at least one chamber.

The provision of a first and a second chamber may allow a user to combine two different odour inhibition substrates to customise their experience. For example, the first odour inhibition substrate may be configured to release odour inhibition molecules having a first fragrance, and the second odour inhibition substrate may be configured to release odour inhibition molecules having a second fragrance. Alternately, the first odour inhibition substrate may be configured to release odour inhibition molecules having a first fragrance, and the second odour inhibition substance may be configured to release odour inhibition molecules which produce an olfactory white.

The aerosol generating device comprises a heater for heating an aerosol-forming substrate. The heater may be configured to heat an aerosol-forming substrate of an aerosol generating article to a temperature sufficient to generate an aerosol.

The heater may be any heater. The heater may be an internal heater configured to be inserted into an aerosol-forming substrate of an aerosol generating article. For example, the heater may be a pin heater, or a blade heater configured to be inserted into an aerosol-forming substrate of an aerosol generating article.

The heater may be an external heater configured to heat an aerosol-forming substrate of an aerosol generating article.

The internal or external heater may be an electrical resistance heater.

The heater may be an induction heater. The induction heater may comprise an induction coil configured to inductively heat a susceptor. The susceptor may be disposed in the aerosol generating device or in an aerosol generating article to be used with the aerosol generating device.

The aerosol generating device may be configured to produce an aerosol from a liquid aerosol-forming substrate. Where this is the case, the heater may comprise a coil-and-wick arrangement.

The aerosol generating device may comprise control electronics for controlling the operation of the aerosol generating device. The aerosol generating device may comprise a user input device to allow a user to activate the aerosol generating device. The user input device may allow a user to activate the heater. The user input device may allow a user to activate the odour inhibition device. The user input device may comprise at least one button.

According to the invention there is also provided an aerosol generating system comprising an aerosol generating device according to the present invention any preceding claim, and at least one odour inhibition substrate disposed in the at least one chamber.

The at least one odour inhibition substrate may be a consumable odour inhibition substrate. The consumable odour inhibition substrate may be configured to release a sufficient concentration of odour inhibition molecule to inhibit the odour associated with consuming one aerosol generating article. This may be advantageous since it may make it easy for a user to determine when they need to replace the odour inhibition substrate. Alternately the odour inhibition substrate may be configured to release a sufficient concentration of odour inhibition molecule to inhibit the odour associated with consuming more than one aerosol generating article. This may advantageously extend the time between replacing odour inhibition substrates.

The aerosol generating system may be configured such that the at least one odour inhibition substrate may need to be replaced following the consumption of at least 2 aerosol generating articles, at least 5 aerosol generating articles, at least 10 aerosol generating articles, or at least 20 aerosol generating articles.

The at least one odour inhibition substrate may provide an indication of when the at least one odour inhibition substrate is consumed and needs to be replaced. For example, the at least one odour inhibition substrate may be configured to change colour when the odour inhibition substrate is consumed and needs to be replaced.

The odour inhibition substrate may comprise at least one of a gel or a solid configured to release at least one odour inhibition molecule.

The odour inhibition substrate may comprise a solid substrate structure configured to adsorb a liquid comprising odour inhibition molecules. The solid substrate structure may comprise at least one of paper, cellulose acetate, expanded foam, or a wire mesh.

The odour inhibition substrate may comprise a solid porous substrate configured to release at least one odour inhibition molecule.

The provision of a solid porous substrate may increase the surface area of the odour inhibition substrate. As set out above, a larger surface area of the odour inhibition substrate may advantageously increase the concentration of odour inhibition molecule entrained in the air passing through the at least one chamber.

The at least one odour inhibition molecule may comprise a masking odour molecule.

The masking odour molecule may have a fragrance which masks or obscures unpleasant odours, such as off-odour and malodour, caused by the heating of the aerosol-forming substrate. The fragrance may be any fragrance. For example, the fragrance may comprise at least one of rose, mint, menthol, chocolate, vanilla, floral, herbal, or spice.

The at odour inhibition molecules may comprise odorant molecules configured to produce an olfactory white. Compounds that produce olfactory white are generally mixtures of multiple odorants (for example, 10 components, 20 components or up to 40 components) that stimulate olfactory perception in a way that creates a perception of a "same" smell.

A human nose comprises an olfactory ephithelium, a strip of tissue comprising millions of sensory neurons. Each sensory neuron is configured to receive only a limited number of types of odour molecule. Without wishing to be bound by theory, it is thought that the particular odour molecules which lead to unpleasant odours, such as off-odour and malodour, caused by the heating of the aerosol-forming substrate may be identified. It may then be possible to provide odour inhibition molecules configured to occupy the same sensory neurons which would be occupied by the molecules which cause the unpleasant odours. Since the relevant sensory neurons are occupied, the user is unable to recognise the unpleasant odours. In doing so, these odour inhibition molecules are said to produce an "olfactory white".

The odour inhibition molecule configured to produce an olfactory white may comprise a molecule which inhibits the interaction of ATP or ATP analogue with a P2X or a P2Y purinergic receptor.

Without wishing to be bound by theory, it may also be the case that exposing the olfactory ephithelium to a plurality of different odour molecules may inhibit a subject's ability to distinguish individual odours. This may be particularly true when the olfactory ephithelium is exposed to a large number of different odour molecules spanning a wide range of different odours. For example, the plurality of odour molecules may comprise at least 10, at least 15, at least 20, or at least 30 different odour molecules spanning a wide range of different odours. It may also be the case that the plurality of different odour molecules need to be present in substantially the same concentration.

The odour inhibition molecule configured to produce an olfactory white may comprise at least one of, for example, abhexone, acetophenone, ortho-acetyl pyridine, strawberry aldehyde, gamma-nonalactone, isoamyl acetate, amyl butyrate, iso-pentyl phenyl acetate, pentyl valerate, anisole, benzaldehyde, iso-bornyl acetate, butanoic acid, butyl sulphide, caryophyllene, celeriax, chlorothymol, cinnamic aldehyde, coumarin, p-cresol, p-cresyl acetate, p-cresyl-iso-butyrate, 4-methyl anisole, cuminic aldehyde, cyclohexnol, 2,4-trans-trans-decadienal, dibutyl amine, diethyl sulphide, dimethyl benzyl carbonyl butyrate, muguet carbinol, 2,3-dimethyl pyrazine, 2,5-dimethyl pyrazine, dimethyl trisulfide, diphenyl oxide, ethyl butyrate, ethyl propionate, 2-ethyl pyrazine, eucalyptol, eugenol, furfuryl mercaptan, guaiacol, heptanal, hexanoic acid, 1-hexanol, 3-hexanol, trans-1-hexanal, 2-phenyl propionaldehyde dimethyl acetal, hydroxyl citronellal, indole, linalool, melanal, 1-menthol, 2-methoxy naphthalene, methyl anthranilate, methyl acetaldehyde dimethyl acetal, para-methyl quinolone, methyl salicylate, S-(methyl thio) butyrate, musk galaxolide, nonyl acetate, 1-octanol, 1-octen 3 - ol, pentanoic acid, 4-pentanoic acid, phenyl acetic acid, phenyl acetylene, phenyl ethanol, iso-phorone, alpha-pinene, propyl butyrate, propyl sulphide, skatole, α-Terpineol, thioglycolic acid, thiophene, thymol, ortho-tolualdehyde, toluene, gamma-undecalactone, undecyclenic acid, iso-valeraldehyde, iso-valeric acid, gamma-valerolactone, vanillin, flower extract, herbal extract, tobacco, tobacco extract, fruit extract, spice, spice extract, or other components. The aerosol generating system may further comprise an aerosol generating article comprising an aerosol-forming substrate, the aerosol generating article arranged to be heated by the heater.

The aerosol generating article comprises an aerosol-forming substrate. The aerosol-forming substrate may be a solid aerosol-forming substrate. Alternatively, the aerosol-forming substrate may comprise both solid and liquid components. The aerosol-forming substrate may comprise a tobacco-containing material containing volatile tobacco flavour compounds, which are released from the substrate upon heating. Alternatively, the aerosol-forming substrate may comprise a non-tobacco material. The aerosol-forming substrate may further comprise one or more aerosol formers. Examples of suitable aerosol formers include, but are not limited to, water, glycerine and propylene glycol.

The aerosol generating article may comprise a liquid aerosol-forming substrate. The liquid aerosol forming substrate may comprise aerosol former. The liquid aerosol-forming substrate may comprise nicotine.

The aerosol-forming substrate may be a tobacco substrate. For example, the aerosol-forming substrate may comprise a rod comprising a tobacco-containing material.

The aerosol generating article may further comprise additional components including, but not limited to, a hollow acetate tube, a transfer element, a cooling element, a filter, or a mouthpiece.

The aerosol generating article may be generally cylindrical and elongate in shape.

The aerosol-forming substrate, and other components of the aerosol generating article, may be connected by a wrapper.

Examples will now be further described with reference to the figures in which:

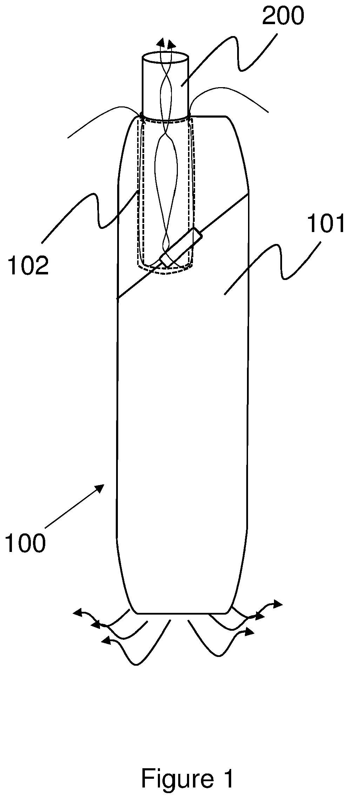

Figure 1 shows a side view of an aerosol generating device according to the present invention.
Figures 2, 3, and 4 show side views of an aerosol generating device according to the present invention with the odour inhibition assembly identified. Figures 2, 3, and 4 show the same aerosol generating device with the odour inhibition assembly at different stages of operation. Figures 2, 3, and 4 also show plan views from the distal end of the odour inhibition assembly at different stages of operation.
Figure 5 shows a detailed view of the odour inhibition assembly used in an aerosol generating device according to the present invention.

The aerosol generating device 100 of the present invention comprises a housing 101 extending between a proximal end and a distal end. A heater (not shown) for heating an aerosol generating article 200 is disposed in a recess 102 at the proximal end of the aerosol generating article 100. The recess 102 is sized to accommodate a single aerosol generating article 200. The heater is a resistively heated blade heater. The aerosol generating device 100 further comprises a battery (not shown) and control electronics (not shown) to supply power to the heater.

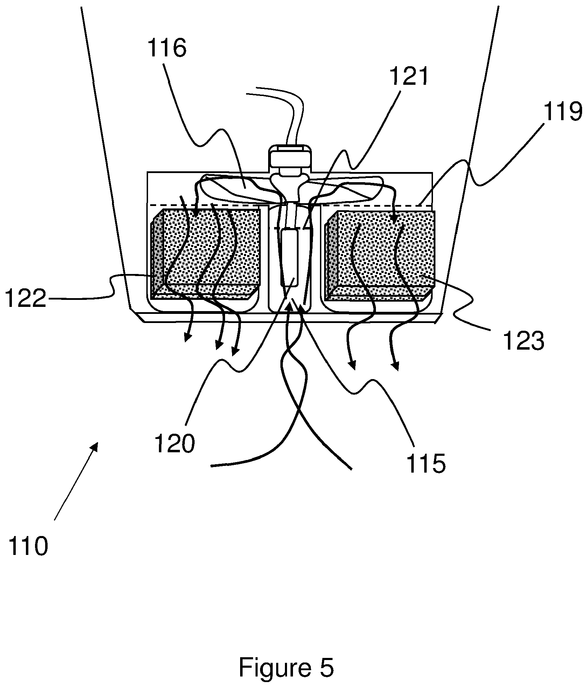

As shown best in Figure 5, the aerosol generating device 100 comprises a charging port 115 disposed at the distal end of the device 100 to allow the device 100 to be connected to an external power supply. The charging port 115 comprises electrical connections 120 to provide an electrical connection to an external power supply.

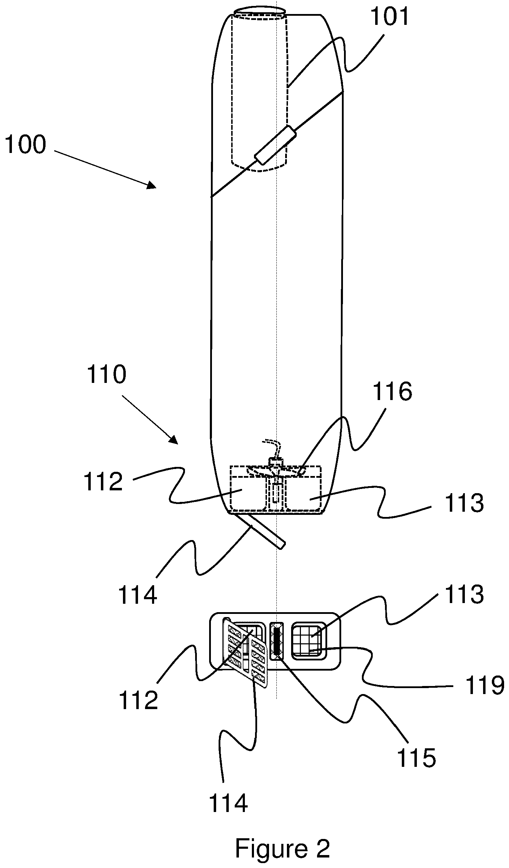

Referring to Figure 2, the aerosol generating device 100 further comprises an odour inhibition assembly 110. The odour inhibition assembly 110 is disposed on the distal end face of the aerosol generating device 100. The odour inhibition assembly 110 comprises a first chamber 112 for receiving a first odour inhibition substrate 122 and a second chamber 113 for receiving a second odour inhibition substrate 123. The first chamber 112 and the second chamber 113 are disposed either side of the charging port 115. The first chamber 112 and the second chamber 113 are each sized to accommodate the first odour inhibition substrate 122 and the second odour inhibition substrate 123 respectively. The first chamber 112 and the second chamber 113 each have a depth of about 5 millimetres.

The charging port 115 further comprises an opening in fluid communication with the first and second chambers 112, 113 to allow air to pass into the odour inhibition assembly 110. In this way, the charging port 115 acts as an air inlet for the odour inhibition arrangement 110.

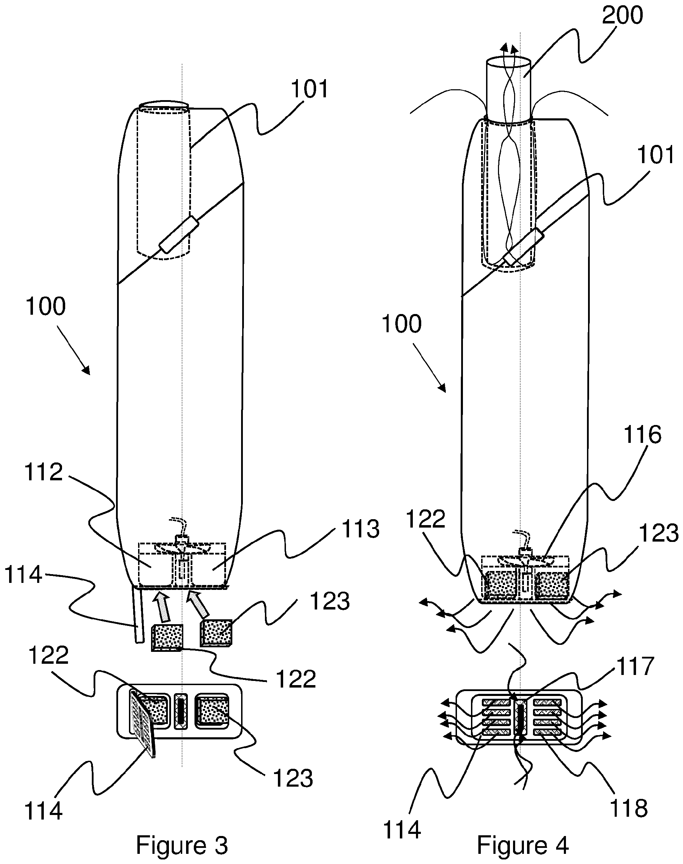

The odour inhibition assembly 110 further comprises a cover 114 connected to the remainder of the aerosol generating device 100 by a hinge connection. The cover 114 is movable between an open position in which the odour inhibition substrates 122, 123 may be inserted and removed from the chambers 112, 113, and a closed position in which the odour inhibition substrates 122, 123 are retained in the chambers 112, 113. The open position is shown in Figure 2 and Figure 3. The closed position is shown in Figure 4.

As shown best in Figure 4, the cover 114 comprises a central opening 117 configured to be aligned with the charging port 115 to allow air to enter the odour inhibition assembly 110. The cover 114 comprises a series of slits 118 either side of the central opening. These slits 118 are configured to be aligned with the first and second the chambers 112, 113 to allow air to leave the odour inhibition assembly. In this way, the slits 118 act as an air outlet for the odour inhibition arrangement 110.

As shown best in Figure 2 and Figure 5, the first and second the chambers 112, 113 each contain a porous support structure in the form of a wire mesh 119 configured to hold the first and second odour inhibition substrates 122, 123 while allowing air to pass around all sides of the odour inhibition substrates 122, 123.

As shown best in Figure 5, the odour inhibition assembly 110 comprises a filter 121 in the form of a mesh disposed within the charging port 115.

The odour inhibition assembly 110 provides an airflow pathway to allow air to enter the odour inhibition assembly through the charging port 115, acting as an air inlet, into the chambers 112, 113 and out through the slits 118, acting as an air outlet.

The odour inhibition assembly 110 further comprises a fan 116 configured to move the air along the airflow pathway from the air inlet to the air outlet. The fan 116 is arranged behind the proximal end of the chambers 112, 113. The fan 116 is a rotational fan comprising a plurality of fan blades arranged to rotate about the longitudinal axis of the aerosol generating device 100. The fan 116 is powered by the battery of the aerosol generating device 100. The activation of the fan 116 is controlled by a user input device (not shown). The fan operates at about 3.3 volts. The fan 116 has a power consumption of about 165 milliwatts. The fan 116 has a diameter of about 10 millimetres. The fan 116 has a depth of about 2 millimetres.

The odour inhibition assembly 110 is configured to be used with a first and second odour inhibition substrate 122, 123. The first and second odour inhibition substrate 122, 123 comprise a solid substrate structure configured to adsorb liquid comprising odour inhibition molecules. The odour inhibition molecules released by both the first and second odour inhibition substrate 122, 123 are configured to produce an olfactory white based on the odours generated by the heating of an aerosol generating article 200.

In use, a user ensures that the cover 114 is in the open position and the first odour inhibition substrate 122 is inserted into the first chamber 112, and the second odour inhibition substrate 123 is inserted into the second chamber 113. This is shown in Figure 3. The cover 114 is then closed.

An aerosol generating article 200 is then inserted into the recess 102 such that the resistively heated blade heater is inserted into an aerosol-forming substrate of the aerosol generating article 200. The aerosol generating device 100 is then activated by a user and the resistively heated blade heater heats the aerosol-forming substrate to generate an aerosol. The user also activates the fan 116 of the odour inhibition assembly 110. This draws air into the odour inhibition assembly 110 through the charging port 115 and into the first and second chambers 112, 113. As the air passes around the first and second odour inhibition substrates 122, 123, the air becomes entrained with odour inhibition molecules before is leaves the odour inhibition assembly through the slits 118. The air entrained with odour inhibition molecules is released into the air surrounding the aerosol generating article to reduce or prevent unpleasant odours, such as off-odour and malodour, caused by the heating of the aerosol-forming substrate.

Once the concentration of odour inhibition molecule released from the odour inhibition substrates 122, 123 is reduced, the odour inhibition substrates 122, 123 are considered to have been consumed. The consumed odour inhibition substrates 122, 123 are then replaced.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10% of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

## Claims

1. An aerosol generating device (100) for use with an aerosol generating article (200) comprising an aerosol-forming substrate, the device comprising:
a heater for heating an aerosol-forming substrate;
an odour inhibition assembly (110), the odour inhibition assembly (110) comprising at least one chamber (112, 113) for receiving at least one odour inhibition substrate (122, 123); and
a recess (102) for receiving an aerosol generating article (200), the at least one chamber (112, 113) being isolated from the recess (102) such that air is unable to pass from the chamber to the recess (102) through the aerosol generating device (100).

2. An aerosol generating device (100) according to claim 1, wherein the odour inhibition assembly (110) is disposed at the distal end of the device.

3. An aerosol generating device (100) according to any preceding claim, wherein the odour inhibition assembly (110) further comprises a cover (114) movable between a closed position, in which the at least one chamber (112, 113) is closed by the cover (114), and an open position, in which the at least one chamber (112, 113) is not closed by the cover (114).

4. An aerosol generating device (100) according to claim 3, wherein the odour inhibition assembly (110) comprises an airflow pathway configured such that air may pass into the assembly though an air inlet, pass through the at least one chamber (112, 113), and pass out of an air outlet.

5. An aerosol generating device (100) according to claim 4, wherein the air outlet is provided through the cover (114).

6. An aerosol generating device (100) according to claim 4 or claim 5, further comprising a charging port (115) for connecting the aerosol generating device (100) to an external power supply, wherein the charging port (115) is in fluid communication with the at least one chamber (112, 113), the charging port (115) acting as the air inlet.

7. An aerosol generating device (100) according to any one of claims 4 to 6, wherein the cover (114) further comprises an opening configured to be aligned with the charging port (115) when the cover (114) is in the closed position.

8. An aerosol generating device (100) according to any one of claims 4 to 7, wherein the odour inhibition assembly (110) further comprises a fan (116) configured to move the air along the airflow pathway.

9. An aerosol generating device (100) according to any preceding claim, wherein the at least one chamber (112, 113) comprises a first chamber (112) for receiving a first odour inhibition substrate (122), and a second chamber (113) for receiving a second odour inhibition substrate (123).

10. An aerosol generating system comprising,
an aerosol generating device (100) according to any preceding claim, and
at least one odour inhibition substrate (122, 123) disposed in the at least one chamber (112, 113).

11. An aerosol generating system according to claim 10, wherein the odour inhibition substrate (122, 123) comprises a solid porous substrate configured to release at least one odour inhibition molecule.

12. An aerosol generating system according to claim 11, wherein the at least one odour inhibition molecule comprises a molecule configured to produce an olfactory white based on the odours generated by the heating of an aerosol generating article (100).

13. An aerosol generating system according to any one of claims 10 to 12, further comprising an aerosol generating article comprising an aerosol-forming substrate, the aerosol generating article arranged to be heated by the heater.

14. An aerosol generating system according to claim 13, wherein the aerosol-forming substrate is a tobacco substrate.

## Patentansprüche

1. Aerosolerzeugungsvorrichtung (100) zum Gebrauch mit einem aerosolerzeugenden Artikel (200), der ein aerosolbildendes Substrat umfasst, die Vorrichtung umfassend:
eine Heizvorrichtung zum Erwärmen eines aerosolbildenden Substrats;
eine Geruchshemmungsbaugruppe (110), wobei die Geruchshemmungsbaugruppe (110) wenigstens eine Kammer (112, 113) zum Aufnehmen wenigstens eines Geruchshemmungssubstrats (122, 123) umfasst; und
eine Aussparung (102) zum Aufnehmen eines aerosolerzeugenden Artikels (200), wobei die wenigstens eine Kammer (112, 113) von der Aussparung (102) isoliert ist, sodass durch die Aerosolerzeugungsvorrichtung (100) keine Luft von der Kammer zu der Aussparung (102) gelangen kann.

2. Aerosolerzeugungsvorrichtung (100) nach Anspruch 1, wobei die Geruchshemmungsbaugruppe (110) an dem distalen Ende der Vorrichtung angeordnet ist.

3. Aerosolerzeugungsvorrichtung (100) nach einem beliebigen vorhergehenden Anspruch, wobei die Geruchshemmungsbaugruppe (110) ferner eine Abdeckung (114) aufweist, die zwischen einer geschlossenen Stellung, in der die wenigstens eine Kammer (112, 113) durch die Abdeckung (114) verschlossen ist, und einer offenen Stellung, in der die wenigstens eine Kammer (112, 113) nicht durch die Abdeckung (114) verschlossen ist, beweglich ist.

4. Aerosolerzeugungsvorrichtung (100) nach Anspruch 3, wobei die Geruchshemmungsbaugruppe (110) einen Luftstromweg aufweist, der ausgelegt ist, dass Luft durch einen Lufteinlass in die Baugruppe eintreten, durch die wenigstens eine Kammer (112, 113) strömen und aus einem Luftauslass austreten kann.

5. Aerosolerzeugungsvorrichtung (100) nach Anspruch 4, wobei der Luftauslass durch die Abdeckung (114) vorgesehen ist.

6. Aerosolerzeugungsvorrichtung (100) nach Anspruch 4 oder Anspruch 5, ferner umfassend einen Ladeanschluss (115) zum Verbinden der Aerosolerzeugungsvorrichtung (100) mit einer externen Energieversorgung, wobei der Ladeanschluss (115) in Fluidverbindung mit der wenigstens einen Kammer (112, 113) steht, wobei der Ladeanschluss (115) als Lufteinlass wirkt.

7. Aerosolerzeugungsvorrichtung (100) nach einem der Ansprüche 4 bis 6, wobei die Abdeckung (114) ferner eine Öffnung aufweist, die ausgelegt ist, mit dem Ladeanschluss (115) ausgerichtet zu sein, wenn sich die Abdeckung (114) in der geschlossenen Stellung befindet.

8. Aerosolerzeugungsvorrichtung (100) nach einem der Ansprüche 4 bis 7, wobei die Geruchshemmungsbaugruppe (110) ferner ein Gebläse (116) umfasst, das zum Bewegen der Luft entlang des Luftstromweges ausgelegt ist.

9. Aerosolerzeugungsvorrichtung (100) nach einem beliebigen vorhergehenden Anspruch, wobei die wenigstens eine Kammer (112, 113) eine erste Kammer (112) zum Aufnehmen eines ersten Geruchshemmungssubstrats (122), und eine zweite Kammer (113) zum Aufnehmen eines zweiten Geruchshemmungssubstrats (123) aufweist.

10. Aerosolerzeugungssystem, umfassend
eine Aerosolerzeugungsvorrichtung (100) nach einem beliebigen vorhergehenden Anspruch; und
wenigstens ein Geruchshemmungssubstrat (122, 123), das in der wenigstens einen Kammer (112, 113) angeordnet ist.

11. Aerosolerzeugungssystem nach Anspruch 10, wobei das Geruchshemmungssubstrat (122, 123) ein festes poröses Substrat umfasst, das zum Freisetzen wenigstens eines Geruchshemmungsmoleküls ausgelegt ist.

12. Aerosolerzeugungssystem nach Anspruch 11, wobei das wenigstens eine Geruchshemmungsmolekül ein Molekül aufweist, das zum Erzeugen eines olfaktorischen Weißes basierend auf den durch das Erwärmen eines aerosolerzeugenden Artikels (100) erzeugten Gerüchen ausgelegt ist.

13. Aerosolerzeugungssystem nach einem der Ansprüche 10 bis 12, ferner umfassend einen aerosolerzeugenden Artikel, der ein aerosolbildendes Substrat umfasst, wobei der aerosolerzeugende Artikel zum Erwärmen durch die Heizvorrichtung angeordnet ist.

14. Aerosolerzeugungssystem nach Anspruch 13, wobei das aerosolbildende Substrat ein Tabaksubstrat ist.

## Revendications

1. Dispositif de génération d'aérosol (100) pour une utilisation avec un article de génération d'aérosol (200) comprenant un substrat formant aérosol, le dispositif comprenant :
un dispositif de chauffage destiné à chauffer un substrat formant aérosol ;
un ensemble d'inhibition d'odeur (110), l'ensemble d'inhibition d'odeur (110) comprenant au moins une chambre (112, 113) pour recevoir au moins un substrat d'inhibition d'odeur (122, 123) ; et
un évidement (102) pour recevoir un article de génération d'aérosol (200), l'au moins une chambre (112, 113) étant isolée de l'évidement (102) de sorte que l'air ne peut pas passer de la chambre à l'évidement (102) à travers le dispositif de génération d'aérosol (100).

2. Dispositif de génération d'aérosol (100) selon la revendication 1, dans lequel l'ensemble d'inhibition d'odeur (110) est disposé au niveau de l'extrémité distale du dispositif.

3. Dispositif de génération d'aérosol (100) selon l'une quelconque des revendications précédentes, dans lequel l'ensemble d'inhibition d'odeur (110) comprend en outre un couvercle (114) mobile entre une position fermée, dans laquelle l'au moins une chambre (112, 113) est fermée par le couvercle (114), et une position ouverte, dans laquelle l'au moins une chambre (112, 113) n'est pas fermée par le couvercle (114).

4. Dispositif de génération d'aérosol (100) selon la revendication 3, dans lequel l'ensemble d'inhibition d'odeur (110) comprend un passage d'écoulement d'air configuré de telle sorte que l'air peut passer dans l'ensemble à travers une entrée d'air, traverser l'au moins une chambre (112, 113) et sortir par une sortie d'air.

5. Dispositif de génération d'aérosol (100) selon la revendication 4, dans lequel la sortie d'air est prévue à travers le couvercle (114).

6. Dispositif de génération d'aérosol (100) selon la revendication 4 ou la revendication 5, comprenant en outre un port de charge (115) pour raccorder le dispositif de génération d'aérosol (100) à une alimentation électrique externe, dans lequel le port de charge (115) est en communication fluidique avec l'au moins une chambre (112, 113), le port de charge (115) agissant comme l'entrée d'air.

7. Dispositif de génération d'aérosol (100) selon l'une quelconque des revendications 4 à 6, dans lequel le couvercle (114) comprend en outre une ouverture configurée pour être alignée avec le port de charge (115) lorsque le couvercle (114) est dans la position fermée.

8. Dispositif de génération d'aérosol (100) selon l'une quelconque des revendications 4 à 7, dans lequel l'ensemble d'inhibition d'odeur (110) comprend en outre un ventilateur (116) configuré pour déplacer l'air le long du passage d'écoulement d'air.

9. Dispositif de génération d'aérosol (100) selon l'une quelconque des revendications précédentes, dans lequel l'au moins une chambre (112, 113) comprend une première chambre (112) pour recevoir un premier substrat d'inhibition d'odeur (122), et une deuxième chambre (113) pour recevoir un deuxième substrat d'inhibition d'odeur (123).

10. Système de génération d'aérosol comprenant,
un dispositif de génération d'aérosol (100) selon l'une quelconque des revendications précédentes, et
au moins un substrat d'inhibition d'odeur (122, 123) disposé dans l'au moins une chambre (112, 113).

11. Système de génération d'aérosol selon la revendication 10, dans lequel le substrat d'inhibition d'odeur (122, 123) comprend un substrat poreux solide configuré pour libérer au moins une molécule d'inhibition d'odeur.

12. Système de génération d'aérosol selon la revendication 11, dans lequel l'au moins une molécule d'inhibition d'odeur comprend une molécule configurée pour produire un blanc olfactif sur la base des odeurs générées par le chauffage d'un article de génération d'aérosol (100).

13. Système de génération d'aérosol selon l'une quelconque des revendications 10 à 12, comprenant en outre un article de génération d'aérosol comprenant un substrat formant aérosol, l'article de génération d'aérosol étant agencé pour être chauffé par le dispositif de chauffage.

14. Système de génération d'aérosol selon la revendication 13, dans lequel le substrat formant aérosol est un substrat de tabac.
